# FASCICULE DE BREVET EUROPEEN

(11) **EP 2 367 525 B1**
(45) Date de publication et mention de la délivrance du brevet: **09.03.2016**
(21) Numéro de dépôt: 09804284.9
(22) Date de dépôt: 22.12.2009
(51) Int. Cl.: A61Q 17/00, A61Q 11/00, A61Q 19/00, A61K 8/97

(54) **COMPOSITION COMPRENANT DES SAPONINES**
ZUSAMMENSETZUNG MIT SAPONINEN
COMPOSITION CONTAINING SAPONINS

(30) Priorité: 23.12.2008 FR 0807412
(43) Date de publication de la demande: 28.09.2011
(73) Titulaire: Universite De Nice-Sophia Antipolis, 06103 Nice Cedex 02 (FR); CENTRE HOSPITALIER UNIVERSITAIRE DE NICE, 06003 Nice Cedex 1 (FR)
(72) Inventeur: MADINIER, Isabelle, F-06000 Nice (FR); GÉRIBALDI, Mireille, F-06100 Nice (FR)
(74) Mandataire: Macquet, Christophe
(86) Numéro de dépôt international: PCT/FR2009/001471
(87) Numéro de publication internationale: WO 2010/072923

(56) Documents cités:
- WEBSTER ET AL: "Antifungal activity of medicinal plant extracts; preliminary screening studies" JOURNAL OF ETHNOPHARMACOLOGY, ELSEVIER SCIENTIFIC PUBLISHERS LTD, IE, vol. 115, no. 1, 28 novembre 2007 (2007-11-28), pages 140-146, XP022368554 ISSN: 0378-8741
- MOREL A F ET AL: "Antimicrobial activity of extractives of Solidago microglossa" FITOTERAPIA, IDB HOLDING, MILAN, IT, vol. 77, no. 6, 1 septembre 2006 (2006-09-01), pages 453-455, XP024925864 ISSN: 0367-326X [extrait le 2006-09-01]
- THIEM B ET AL: "Antimicrobial activity of Solidago virgaurea L. from in vitro cultures" MEDICINAL & AROMATIC PLANTS ABSTRACTS, RESOURCES, NEW DELHI, INDIA - NEW DELHI, vol. 25, no. 3, 1 juin 2003 (2003-06-01), XP018016668 ISSN: 0250-4367
- DUARTE M C T ET AL: "Anti-Candida activity of Brazilian medicinal plants" JOURNAL OF ETHNOPHARMACOLOGY, ELSEVIER SCIENTIFIC PUBLISHERS LTD, IE, vol. 97, no. 2, 28 février 2005 (2005-02-28), pages 305-311, XP025269767 ISSN: 0378-8741 [extrait le 2005-02-28]
- REZNICEK,JURENITSCH,KUBELKA,MICHL,KORHAMME R: "Isolation and structure of the four main saponins from Soligado gigantea var. serotina" LIEBIGS ANNALEN DER CHEMIE, vol. 10, 1990, pages 989-994, XP002544958
- PEPELJNJAK,KUSTRAK,VUKUSIC: "Investigation of the antimycotic activity of Soligado virgaurea and Soligado gigantea extracts" PHARMACEUTICAL PHARMACOLOGICAL LETTERS, vol. 2, 1998, pages 85-86, XP009122511
- DATABASE WPI Week 199235 Thomson Scientific, London, GB; AN 1992-286776 XP002438015 & HU 59 815 A (BIOGAL GYOGYSZERGYAR) 28 juillet 1992 (1992-07-28)

## Description

La présente invention concerne une composition comprenant des saponines. Cette composition est particulièrement utile comme bain de bouche, notamment chez des individus présentant une sécheresse buccale. Elle est également utile pour l'hygiène de la peau, des muqueuses, des ongles et/ou des cheveux, dans le traitement des sécheresses cutanées et/ou des sécheresses des muqueuses, et dans le traitement des mycoses, en particulier cutanées ou génitales.

Actuellement, les bains de bouche sont utilisés comme antiseptiques de la bouche, comme produits d'usage régulier pour l'hygiène bucco-dentaire quotidienne, ou en cas d'opération chirurgicale ou d'infections bucco-dentaires.

Les bains de bouche connus, dits antiseptiques, contiennent divers ingrédients, notamment des antiseptiques à large spectre, des détergents de synthèse ou des principes actifs agissant contre les symptômes des infections ou inflammations bucco-dentaires. Les détergents et les antiseptiques, par exemple l'éthanol, le phénol, l'héxétidine, la chlorhexidine et les ammoniums quaternaires, inhibent le développement ou détruisent les bactéries du biofilm oral et les *Candida*, et notamment *C*. *albicans* (*Candida albicans)* qui est le champignon pathogène majeur du biofilm et souvent associé à la bouche sèche. Ces bains de bouche sont également utilisés en cas de sécheresse buccale.

Pourtant, ces bains de bouche antiseptiques présentent des inconvénients majeurs. En effet, de manière surprenante, grâce à deux études cliniques, les Demanderesses ont mis en évidence que leur usage régulier est une cause de sécheresse buccale. Or, la sécheresse buccale favorise les candidoses orales et les caries dentaires. Les Demanderesses ont également mis en évidence une association entre la sécheresse buccale et la colonisation des prothèses dentaires par des champignons.

Les bains de bouche antiseptiques de l'état de la technique ne conviennent que dans des indications très limitées, en pré- et post-opératoire, et lors d'infections aiguës des muqueuses. Au contraire, ils ne sont pas adaptés pour une utilisation régulière, de longue durée. Pourtant, les Demanderesses ont mis en évidence que leur utilisation, qui ne doit jamais dépasser deux semaines est souvent beaucoup plus longue, voire quotidienne sans limitation de durée. Une utilisation prolongée déséquilibre le biofilm, notamment le biofilm oral. En particulier, elles ont observé un usage iatrogène supérieur à deux semaines chez 34% de 90 malades chroniques (âge moyen 44 ans), et chez 27% de 60 personnes âgées hospitalisées (âge moyen 85 ans). En particulier, les Demanderesses ont identifié que le lien entre la sécheresse buccale et les bains de bouche antiseptiques connus vient de ce que ces bains de bouche contiennent des principes actifs qui détruisent en même temps les bactéries du biofilm sain bucco-dentaire et qui dénaturent les polysaccharides de ce biofilm sain, y compris les polysaccharides des glycoprotéines. Or, ces polysaccharides ont la propriété de fixer un grand nombre de molécules d'eau, et ils assurent l'hydratation et la viscosité du biofilm.

Les Demanderesses ont identifié que les principes actifs antimicrobiens utilisés dans les bains de bouche sont essentiellement l'éthanol, les tanins, les ammoniums quaternaires, les bisguanides, les huiles essentielles et les ions bicarbonates, seuls ou en combinaison.

Les Demanderesses concluent également de ces études que le phénomène de bouche sèche induit par les bains de bouche antiseptiques et autres médicaments est auto-entretenu par ces traitements. En effet, la prolifération des *Candida*, favorisée par le manque de (protéo) glycanes physiologiques, provoque une acidification du milieu buccal. Les *Candida* produisent alors des protéases actives en milieu acide, qui hydrolysent la partie protéique des mucines et des protéines riches en proline de la salive et du biofilm oral. La prolifération des *Candida* provoque et entretient une inflammation des muqueuses avec augmentation de la perméabilité des muqueuses aux médicaments. Au total, l'association bains de bouche antiseptiques et candidose orale détruit les glycoprotéines du biofilm oral par une attaque simultanée des glucides (antiseptiques) et des protéines (protéases des *Candida*)*.*

Des traitements antifongiques existent en cas de candidose orale, notamment les polyènes et les azolés. Cependant, les *Candida* développent des résistances vis-à-vis de ces traitements.

Aussi, il n'existe pas de traitement antifongique qui soit respectueux du biofilm sain de la zone traitée et sans effet secondaire néfaste pour la zone traitée.

Le problème que se propose de résoudre l'invention est d'obtenir une composition qui ne présente pas les inconvénients associés aux bains de bouche de l'état de la technique, et en particulier qui puisse être utilisée de manière régulière et prolongée sans provoquer de sécheresse buccale, qui soit efficace contre les candidoses orales. Plus largement, l'invention se propose de fournir une nouvelle composition qui permette de lutter contre le développement des candidoses, en particulier orales, cutanées et génitales, et/ou contre les sécheresses buccales, dans le cadre d'une hygiène régulière ou de traitements pharmaceutiques ou dermatologiques prolongés, peu coûteux.

La solution proposée de l'invention à ce problème posé a pour premier objet une composition comprenant un extrait d'une plante solidage *(Solidago)* comprenant des saponines et substantiellement exempte de tanins, les saponines n'ayant pas subi de lyse alcaline dans le cas où l'extrait de plante consiste en des saponines purifiées.

De manière avantageuse, - la composition ne comprend pas de tanins; - la composition se présente sous une forme de bain de bouche, dentifrice, gel, crème, lait, poudre, lotion, spray, savon ou ovules, et de préférence sous une forme de bain de bouche.

L'invention a pour deuxième objet l'utilisation cosmétique d'une composition comprenant un extrait d'une plante solidage *(Solidago)* comprenant des saponines et ne comprenant substantiellement pas de tanins pour l'hygiène de la bouche, de la peau, des muqueuses, des ongles et/ou des cheveux.

L'invention a également pour objet une composition comprenant un extrait d'une plante solidage *(Solidago)* comprenant des saponines et ne comprenant substantiellement pas de tanins pour son application comme médicament.

L'invention a également pour objet une composition comprenant un extrait d'une plante solidage *(Solidago)* comprenant des saponines et ne comprenant substantiellement pas de tanins pour son application comme médicament dans le traitement des sécheresses cutanées et/ou des sécheresses des muqueuses, et en particulier des sécheresses buccales.

L'invention a également pour objet une composition comprenant un extrait d'une plante solidage *(Solidago)* comprenant des saponines et ne comprenant substantiellement pas de tanins pour son application comme médicament dans le traitement des mycoses, en particulier des mycoses cutanées, buccales ou ano-génitales.

L'invention a également pour objet une composition comprenant un extrait d'une plante solidage *(Solidago)* comprenant des saponines et ne comprenant substantiellement pas de tanins pour son application comme médicament dans le traitement des infections bucco-dentaires, notamment les caries, les parodontites, la langue noire et l'halithose liées à la sécheresse buccale.

L'invention a également pour objet un procédé d'obtention d'un extrait de plantes, de préférence solidages *(Solidago),* ledit extrait comprenant des saponines et ne comprenant substantiellement pas de tanins, le procédé comprenant les étapes suivantes :
- d'extraction de saponines à partir de plantes, de préférence solidages (*Solidago*) ; puis - d'élimination ciblée des tanins.

De manière avantageuse, - l'étape d'extraction est une extraction aqueuse, éthanolique, méthanolique, une extraction au chloroforme ou encore une extraction à l'éther de pétrole; - l'étape d'extraction, dans le cas d'une extraction éthanolique, méthanolique, au chloroforme ou à l'éther de pétrole, est suivie d'une étape d'élimination de l'éthanol, du méthanol, du chloroforme ou de l'éther de pétrole, par exemple par évaporation ; - l'étape d'élimination ciblée des tanins est effectuée par précipitation des tanins en présence de protéines, et de préférence en présence de caséine.

L'invention sera mieux comprise à la lecture de la description non limitative qui suit et des dessins qui l'accompagnent, dans lesquels :
- la figure 1 montre une vue au microscope de la souche de référence *C*. *albicans* 4872 en culture sur une gélose ; à droite, la culture a été en contact avec un extrait de plante comprenant des saponines selon l'invention ; à gauche, la culture a été en contact avec un milieu sans saponines ;
- la figure 2 montre une vue au microscope de la souche sauvage *C. albicans* MG 001 en culture sur une gélose ; à droite, la culture a été en contact avec un extrait de plante comprenant des saponines selon l'invention ; à gauche, la culture a été en contact avec un milieu sans saponines ;
- la figure 3 montre une vue au microscope de l'effet de différents extraits aqueux de solidages comprenant des saponines sur la transformation des levures de la souche *C**.** albicans* ATCC 10231r (forme rugeuse) ; et
- la figure 4 montre l'effet de différents extraits aqueux de solidages comprenant des saponines sur la taille des colonies de *C. albicans* ATCC 10231r en culture sur une gélose.

La composition selon l'invention présente les avantages suivants :
- elle a un effet antifongique ciblé sur les *Candida,* en particulier *C. albicans* (*Candida albicans*)*,* tout en préservant les bactéries du biofilm sain, notamment de la bouche, de la peau et des muqueuses ano-génitales ;
- elle crée *in vivo* un environnement défavorable à la croissance des *Candida,* sans les éradiquer mais en les maintenant sous une forme quiescente, non virulente ;
- elle préserve les glycanes, les protéines et les glycoprotéines, notamment dans la bouche et sur les muqueuses, par exemple d'origine salivaire et bactérienne. Ainsi, les bactéries et les protéines et glycoprotéines du biofilm sain étant préservées, elles s'opposent également à l'invasion des tissus par les *Candida* et les bactéries pathogènes. En créant un environnement défavorable à la croissance des *Candida*, la composition favorise les bactéries endogènes, car il existe une compétition dans le biofilm muqueux entre les bactéries endogènes et les *Candida ;*
- en préservant les glycanes et glycoprotéines, elle réduit le développement de sécheresses buccales, puisque les glycanes et glycoprotéines contribuent également à l'hydratation et à la viscosité du biofilm, dans la bouche et sur les muqueuses ;
- elle a ainsi une action rééquilibrante sur le biofilm sain de la zone d'application, notamment le biofolm oral, cutané ou uro-génital ;
- elle apaise et hydrate les peaux sensibles et/ou fragiles et elle n'agresse pas les peaux et muqueuses enflammées ;
- dans la bouche, elle permet de maintenir un biofilm peu épais, à moins d'un mm d'épaisseur, limitant ainsi les caries et les parodontites, et permettant l'accés de ses principes actifs à l'ensemble du biofilm.
- elle peut être utilisée de façon régulière et prolongée, sans effets secondaires néfastes, grâce à son action douce, ciblée et différée sur les *Candida ;*
- elle respecte l'environnement car elle ne comprend de préférence ni conservateur, ni colorant, ni parfum de synthèse et elle est biodégradable ;
- elle ne colore pas la zone traitée, notamment la langue, les dents ou les prothèses lors d'une application buccale, et elle n'altère pas le goût chez l'utilisateur lors d'une application buccale ;
- elle ne comprend de préférence pas les terpènes volatiles contenus dans les huiles essentielles, lesquels sont toxiques, et n'induit donc pas de risques de confusion des idées ou d'agitation chez les utilisateurs.

Les tanins sont reponsables d'une précipiation des glycopréotéines du biofilm oral, en particulier des protéines riches en proline. De manière surprenante, les Demanderesses ont identifié que les tanins compris dans les compositions de l'état de la technique ont un effet aggravant sur la sensation de bouche sèche, à cause de leur effet sur le biofilm associé à la bouche sèche. Ainsi, selon une caractéristique essentielle, la composition selon l'invention est substantiellement exempte de tanins, c'est-à-dire ne comprend pas de tanins, ou seulement à l'état de traces. Pour cela, les tanins sont éliminés de manière ciblée de l'extrait de plante. Ainsi, l'absence de tanins semble jouer un rôle majeur dans les avantages des compositions selon l'invention.

Selon une autre caractéristique, la composition selon l'invention comprend des saponines et, de préférence, un extrait d'une plante solidage les comprenant.

Les saponines, également appelées saponosides ou glycosides de saponine, constituent un vaste groupe d'hétérosides complexes appartenant aux terpènes cycliques ou aux stéroïdes. Une saponine est issue de la combinaison chimique d'un sucre (glucide) et d'un stéroïde, d'un stéroide alcaloïde ou d'un triterpène. Certaines compositions de l'art antérieur comprennent des saponines stéroïdiennes ou triterpéniques. Dans le cadre de l'invention, les saponines sont préférentiellement non stéroïdiennes.

Douées de propriétés tensioactives, les saponines font mousser leurs solutions et servent de détergent, par exemple de savons végétaux. Elles ont été également proposées pour traiter l'hypercholestérolémie, comme anticancéreux ou comme adjuvants dans les vaccins. Les saponines sont détergentes, hémolytiques, chélatrices du fer, du zinc et du calcium. Les saponines se fixent sur les stérols membranaires, l'ergostérol des cellules fongiques et le cholestérol des cellules humaines. Comme les bactéries n'ont pas de stérols membranaires, les saponines n'ont pas de propriétés antibactériennes aux doses pharmacologiques. Elles peuvent en avoir toutefois à des doses plus élevées, en tant que détergents.

Les saponines sont présentes chez de nombreux végétaux. Dans le cadre de l'invention, les saponines sont extraites de plante(s) solidage(s). Elles peuvent être extraites des sommités fleuries, feuilles et/ou des rhizomes de plante. De préférence, la plante solidage, encore appelé verge d'or, c'est-à-dire du genre *S*. *(Solidago) L. Asteracea,* peut être choisie parmi l'une des 120 espèces connues, dont *S. virgaurea, S. canadensis, S. microglossa,* et de préférence *S. virgaurea* L. *virgaurea* ou *S. virgaurea* L. *minuta*/*alpestris*.

Les solidages sont cultivés comme plantes médicinales. Par exemple, *S. virgaurea* est présentée comme diurétique, antalgique, anti-inflammatoire, antitumorale, anti-aphtes et anti-ulcères, antiseptique et antidiarrhéique. L'infusion de solidage est traditionnellement utilisée contre les inflammations, les troubles digestifs et urinaires et les aphtes. Cependant, les compositions connues à base de solidages comprennent des tanins, nocifs pour le biofilm.

Plusieurs études de l'art antérieur ont recherché les effets antimicrobiens des divers extraits de solidages et montrent une efficacité peu convaincante contre les *Candida* et/ou une activité antibactérienne que la composition selon l'invention cherche à éviter. Il s'agit dans ces études d'extraits dont les tanins n'ont pas été éliminés.

Par exemple, un extrait aqueux total de *Solidago gigantea* Ait. a été testé contre 13 souches fongiques. Aucune inhibition n'a été observée pour 3 souches de *C*. *albicans* sur 4 ni pour la souche de *C. parapsilosis* testée (Webster J Ethnopharmacol 2008). Par conséquent, ces données dissuadent d'utiliser un extrait aqueux de solidage contre les *Candida*.

Des extraits issus d'une extraction méthanolique de *S. microglossa* ont montré une faible activité antibactérienne ainsi qu'une activité antifongique contre une souche de *C*. *albicans* (Morel, Fitoterapia 2006). Par ailleurs, des extraits des parties aériennes de *S. virgaurea* L. montrent une activité inhibitrice sur 9 souches bactériennes (Thiem, Fitoterapia 2002). Ces études dissuadent d'utiliser un extrait issu d'une extraction méthanolique de solidage pour préserver les bactéries du biofilm sain.

Des extraits issus d'une extraction éthanolique de *S. gigantea* Ait. et de *S*. *virgaurea* L. ont, respectivement, été actifs contre une souche de *C.* (*Candida*) *pseudotropicalis* et sans effet contre 11 souches de *Candida* testées (Pepeljnjak, Pharmaceutical and Pharmacological Letters 1998). Des extraits des feuilles et/ou des racines de *S. chilensis* étaient inefficaces à toutes les concentrations testées contre une souche de *C*. *albicans* (Teixeira Duarte, J Ethnopharmacol 2005). Ces données dissuadent d'utiliser un extrait issu d'une extraction éthanolique de solidage contre les *Candida.*

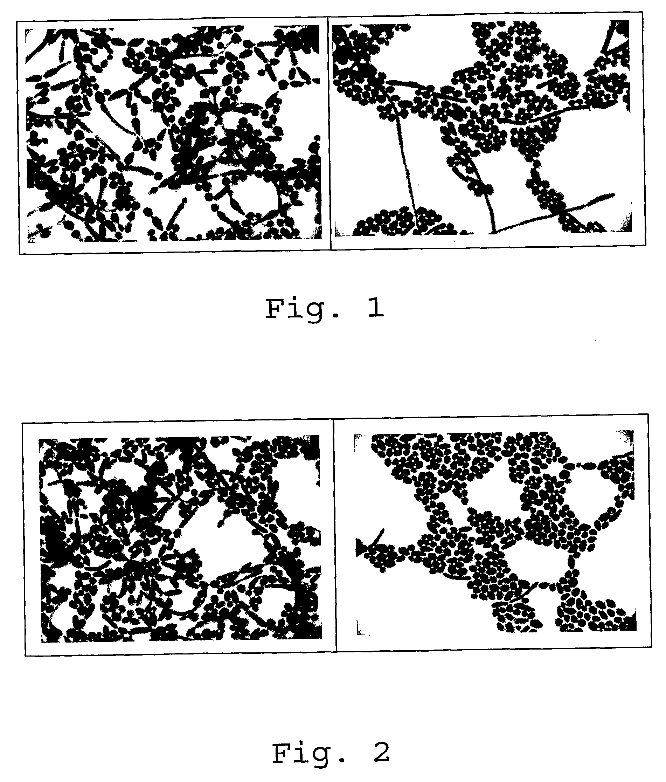

De manière surprenante, dans le cadre de l'invention, les Demanderesses ont mis en évidence que les saponines exercent leur effet antifongique non pas par une action fongicide ou fongistatique comme les compositions de l'art antérieur, mais de manière plus douce et progressive en créant un environnement défavorable à la croissance des *Candida.* Les Demanderesses ont observé que les saponines selon l'invention, sur une culture de *Candida,* ont pour effet non pas de diminuer le nombre de colonies (ou CFU, pour « Colony Forming Units »), mais de diminuer significativement le diamètre des colonies et de modifier la morphologie des *Candida.* Cela a été observé, en particulier, avec une décoction, *i.e*. une infusion ou une extraction aqueuse, de solidage (voir Tableaux 1 à 3 et Figures 1 à 4).

Ainsi, les compositions selon l'invention ont un effet antifongique retard, plus doux et mieux toléré par le biofilm de la zone d'application. Cela peut s'expliquer par le fait que la saponine, détergent non ionique, limite l'apport en lipides, dont les besoins sont très augmentés lors de la transformation des levures *Candida* en filaments. De plus, les saponines sont hémolytiques et chélateurs du fer. Elles privent en fer les *Candida* et les bactéries pathogènes hémophiles du biofilm, notamment oral, notamment les streptocoques cariogènes, les *Haemophilus* et les bacilles Gram-négatifs associés aux parodontites et à l'halithose.

L'extrait de plante comprenant des saponines est de préférence un extrait aqueux. Il peut être issu d'une extraction aqueuse, éthanolique, méthanolique, une extraction au chloroforme ou à l'éther de pétrole.

L'extrait de plante selon l'invention n'est pas une huile essentielle. En effet, les saponines, substances non volatiles, ne sont pas apportées, dans les compositions de l'invention, par des huiles essentielles.

Des études de l'art antérieur ont également été réalisées avec des extraits de saponines purifiées, c'est-à-dire des extraits de plantes à partir desquels les saponines ont été isolées, séparées des autres composants des extraits. Ainsi, un mélange de saponines purifiées de *S. virgaurea* obtenu par extraction éthanolique s'est montré inactif contre 7 souches de *C. albicans* testées (Bader, Pharmazie 1987). Seules les saponines ayant subi une lyse alcaline partielle avaient des propriétés antifongiques, avec un diamètre d'inhibition mesurable contre plusieurs souches de *C. albicans* dans les tests de diffusion sur gélose. Les saponines lysées et purifiées étaient généralement plus actives individuellement que le mélange de saponines lysées (Bader Pharmazie 1987, Bader Pharmazie 1990, Bader 210th National Meeting Chicago 1995, Bader Pharmazie 2000). Ces saponines purifiées étaient des glycosides de l'acide polygalacique, avec un disaccharide et un oligosaccharide de 4 à 8 résidus liés sur l'aglycone. La lyse raccourcit les saccharides qui composent les saponines, et l'intensité de l'effet antifongique de ces saponines purifiées est apparue augmentée lorsque la longueur de l'oligosaccharide qui les compose est raccourcie (Hiller Pharmazie 1987).

Pourtant, selon une caractéristique préférée de l'invention, la ou les saponines n'ont pas subi de lyse alcaline, et en particulier lorsque la ou les saponines de la composition selon l'invention sont purifiées, c'est-à-dire lorsqu'elles sont utilisées dans la composition après avoir été séparées des autres composants de l'extrait végétal dont elles proviennent. En effet, de manière surprenante, dans ces conditions, les saponines présentent l'effet antifongique recherché, selon lequel elles n'ont pas d'effet inhibiteur visible mais s'opposent à la conversion des champignons d'un état quiescent vers un état actif, en ralentissant ou s'opposant à la filamentation. Cette observation des Demanderesses va à l'encontre du préjugé selon lequel seules les saponines purifiées ayant subit une lyse alcaline ont une action contre les *Candida* et selon lequels l'effet antifongique de la saponine est améliorée après une lyse alcaline. Lorsque les saponines de la composition ne sont pas purifiées, c'est-à-dire lorsqu'elles sont utilisées dans la composition avec les autres composants de l'extrait de plantes, à l'exception des tanins, alors elles peuvent avoir ou ne pas avoir subi de lyse alcaline. De préférence, elles n'ont pas subi de lyse alcaline.

Les compositions comprenant un extrait de plante, substantiellement sans tanins mais avec les autres composants de l'extrait, sont préférées car ces autres composants ont des propriétés intéressantes. Ils comprennent notamment des arômes et des colorants naturels et non toxiques.

Les compositions selon l'invention se présentent de préférence sous une forme adaptée pour une application topique, en particulier pour une application bucco-dentaire, cutanée ou génitale. De préférence, les compositions se présentent sous forme de :
- bain de bouche, dentifrice, gel, crème et autres formes galéniques adaptées pour une application bucco-dentaire ;
- bain de trempage, solution, pastille et autres formes galéniques adaptées pour la décontamination ou le nettoyage des prothèses dentaires ou d'autres dispositifs amovibles utilisés notamment en odontologie, en stomatologie ou en chirurgie maxillo-faciale ;
- gel, crème, lait, poudre, lotion, spray, savon et autres formes galéniques adaptés pour une application sur la peau, les ongles et/ou les cheveux ;
- gel, crème, lait, ovule, savon et autres formes galéniques dans des médicaments adaptés pour une application ano-génitale.

L'extrait pur, également appelé solution mère, correspond à l'extrait de plante après l'élimination des tanins, éventuellement après élimination des composants toxiques éventuellement utilisés pour l'extraction.

Les compositions selon l'invention, et en particulier les bains de bouche, comprennent de préférence entre 0,005 et 50 % d'extrait (ou solution mère), et de préférence entre 1 et 15 %, par exemple 10 % en volume du volume total de la composition.

L'invention concerne également les utilisations des compositions selon l'invention.

Les compositions selon l'invention peuvent être utilisées pour l'hygiène bucco-dentaire, de façon régulière, ou bien dans le cadre d'un traitement des candidoses buccales, des sécheresses buccales, des caries, des parodontites, de la langue noire et/ou de l'halithose. Elles sont particulièrement indiquées sous forme de bain de bouche.

Les compositions selon l'invention peuvent également être utilisées, de façon régulière, pour l'hygiène de la peau, des ongles et/ou des cheveux, des muqueuses ano-génitales, notamment en pédiatrie ou en gynécologie. Elles peuvent également être utilisées dans le cadre d'un traitement des mycoses ou des candidoses cutanées, unguéales, capillaires ou ano-génitales.

Les compositions selon l'invention sont de préférence utilisées en tant que médicament complémentaire d'un médicament conventionnel de l'affection concernée.

Les compositions selon l'invention peuvent encore être destinées à un usage vétérinaire dans les indications ci-dessus, sauf chez les animaux à sang froid pour lesquels les saponines sont toxiques.

Les saponines sont présentes dans la plante sous forme de mélanges complexes. L'extraction et surtout la séparation des saponines sont délicates à cause de leur forte polarité, de leur relative fragilité et des très faibles différences structurales entre les constituants.

Les Demanderesses ont mis au point un procédé d'obtention d'un extrait de plantes, en particulier à partir de plantes solidages, ledit extrait comprenant des saponines. Ce procédé est avantageux en ce qu'il permet d'obtenir des saponines n'ayant pas subi de lyse alcaline ainsi qu'un extrait dépourvus de tanins.

Ce procédé comprend de manière essentielle une étape d'extraction de différents composés dont des saponines à partir de plantes ainsi qu'une étape d'élimination ciblée des tanins.

L'étape d'extraction peut comprendre une extraction aqueuse, éthanolique, méthanolique, une extraction au chloroforme ou encore une extraction à l'éther de pétrole.

Lorsque l'extraction est faite avec de l'éthanol, du méthanol, du chloroforme ou de l'éther de pétrole, elle comprend de préférence une étape d'élimination de l'éthanol, du méthanol, du chloroforme ou de l'éther de pétrole, par exemple par évaporation.

L'extraction aqueuse est avantageuse car elle ne nécessite pas une étape d'élimination de l'éthanol, du méthanol, du chloroforme ou de l'éther de pétrole. Elle est plus rapide, plus facile et moins coûteuse.

Le procédé comprend en outre une étape d'élimination ciblée des tanins. Cette étape est avantageusement effectuée par précipitation des tanins en présence de protéines, et de préférence en présence de caséine. En effet, la caséine est avantageuse car elle présente des homologies de séquence avec les protéines majeures du biofilm sain, avec des (glyco)protéines protectrices du biofilm oral sain ainsi qu'avec des protéines salivaires. La caséine permet d'éliminer les tanins ayant la plus forte affinité pour les protéines du biofilm sain.

Un mode de réalisation de procédé d'extraction aqueuse à partir d'une plante, de préférence solidage, selon l'invention comprend les étapes suivantes :
- de préparation d'une décoction, étape selon laquelle on ajoute de l'eau distillée stérile à une quantité de plante (sommités fleuries, feuilles et/ou rhizomes), on porte le mélange à ébullition, on laisse le mélange revenir à température ambiante, puis on le filtre;
- de préparation d'une décoction isotonique, étape selon laquelle on dilue par deux la décoction obtenue à l'étape précédente avec une solution de chlorure de sodium à 1,8% ;
- d'élimination des tanins, étape selon laquelle la décoction isotonique est acidifiée à pH 4 puis de la caséine y est ajoutée afin de former des complexes tanins-caséine. Après une agitation douce, ces complexes sont éliminés par centrifugation et le surnageant est récupéré.

Un mode de réalisation plus particulier d'un procédé d'extraction aqueuse à partir de plante, de préférence solidage, comprend les étapes suivantes:
1) Préparation de la décoction :
   Une quantité de 6g de solidage (*Solidago virgaurea virgaurea L*.), sommités fleuries et feuilles sèches et broyées, est placée dans un bécher stérile de 250 mL dans lequel on ajoute de l'eau distillée stérile à température ambiante (q.s.p. 100 mL). Le bécher est recouvert d'un papier d'aluminium stérile. Il est placé dans un cristallisoir d'eau à température ambiante. L'ensemble est recouvert d'un papier d'aluminium et placé sur une plaque chauffante. Après 10 mn d'ébullition, le bécher est placé sur la paillasse toute la nuit. La décoction revenue à température ambiante présente un pH de 5,49. Elle est filtrée sur un buchner autoclavé équipé d'un papier filtre autoclavé dans une ambiance stérile (sous hotte à flux laminaire). La solution est ensuite clarifiée par centrifugation en tubes stériles et/ou si nécessaire par filtration en milieu stérile. A ce stade, la décoction peut être aliquotée et congelée à -20°C.
2) Décoction isotonique (physiologique) :
   Une décoction isotonique est obtenue en diluant par deux la décoction proprement dite, obtenue à l'étape précédente, par une solution saline de chlorure de sodium stérile à 1,8% de manière à avoir une décoction finale isotonique à 0,9% de chlorure de sodium.
3) Elimination ciblée des tanins :
   La décoction isotonique est acidifiée à pH 4, par exemple par de l'acide citrique. Après acidification de 750 *µ*L de décoction, le volume final est de 760 *µ*L. Le mélange décoction-acide citrique donne un volume final de 760 *µ*L dans un tube eppendorff de 1,5 mL. De la caséine est ajoutée au mélange, qui est soumis à une agitation douce pendant 1h à température ambiante. La caséine utilisée peut être, par exemple, du caséinate de calcium lécithiné alimentaire, titré à 87% de protéines au minimum (Protilight IP4, Armor Protéines, Saint Brice en Coglès, France). Il se forme un complexe tanins-caséine, de couleur brune, visible macroscopiquement et qui précipite. La précipitation maximum de tanins semble située à 3,5 mg de caséine pour 760 *µ*L de décoction. Le complexe est éliminé par centrifugation pendant 10 mn à 10000 g à 20°C. Le surnageant, désigné « solution mère » dans la suite du texte, constitue l'extrait, la décoction de solidage après une élimination ciblée des tanins, et il présente une coloration plus claire. Il peut être congelé à -20°C pour une utilisation ultérieure.
4) Vérification de la présence de saponines dans la solution mère :
   La présence de saponines dans la solution mère est vérifiée par son pouvoir hémolytique, par exemple par diffusion en puits sur gélose. Un volume de 50*µ*L de solution mère est déposé dans un puits de 5mm de diamètre fait dans une gélose comprenant du sang, par exemple une gélose au sang de mouton 5% Columbia^{®}, BioRad^{®} (Marnes-la-Coquette, France). La boîte de gélose est incubée à 37°C et le diamètre d'hémolyse est mesuré toutes les heures. La solution mère utilisée pour les tests microbiologiques donne un diamètre d'hémolyse de 8mm après 1h, 9mm après 2h, 10mm après 3h 10,5mm après 4h, et 10,6mm après 5h et 6h.
5) Concentration de la décoction :
   Pour faciliter les manipulations, la solution mère peut être concentrée, de préférence en utilisant des membranes filtrantes, par exemple par ultrafiltration.

Un autre mode de réalisation plus particulier d'un procédé d'extraction aqueuse à partir de plante, de préférence solidage, comprend les étapes suivantes :
1) Préparation de la décoction :
   Une quantité de 6g des parties aériennes de solidage (*Solidago virgaurea minuta*/*alpestris* (*Walds.* & *Kit.*))*,* séchées, est placée dans un bécher stérile de 250 mL dans lequel on ajoute de l'eau distillée stérile à température ambiante (q.s.p. 100 mL). Le bécher est recouvert d'un papier d'aluminium stérile. Il est placé dans un cristallisoir d'eau à température ambiante. L'ensemble est recouvert d'un papier d'aluminium et placé sur une plaque chauffante. Après 10 mn à 100°C, le bécher est placé sur la paillasse toute la nuit à température ambiante. La décoction est filtrée sur un buchner autoclavé équipé d'un papier filtre autoclavé dans une ambiance stérile (sous hotte à flux laminaire). La solution est ensuite clarifiée par centrifugation à 2000g minimum pendant 15 minutes à 20°C. A ce stade, la décoction peut être aliquotée et congelée à -20°C.
2) Elimination ciblée des tanins :
   Les tanins sont précipités par l'ajout de caséinate de calcium lécithiné alimentaire (5 mg, Protilight IP4^{®}, Armor Protéines, Saint Brice en Coglès, France) et acidifiée à pH 4 avec 25 *µ*L de tampon citrate 1M par mL de décoction. Après 60 minutes d'incubation à température ambiante, et centrifugation à 2000g minimum pendant 15 minutes à 20°C, le surnageant, désigné « solution mère » dans la suite du texte, constitue l'extrait, la décoction de solidage après une élimination ciblée des tanins, est autoclavée et peut être congelée à -20°C pour une utilisation ultérieure.
3) Décoction isotonique (physiologique) :
   Une décoction isotonique peut être obtenue par l'ajout de chlorure de sodium de manière à obtenir 0,9% final.
4) Vérification de la présence de saponines dans la solution mère :
   La présence de saponines dans la solution mère isotonique est vérifiée par son pouvoir hémolytique, par exemple par diffusion en puits sur gélose. Un volume de 50/*µ*L de solution mère isotonique est déposé dans un puits de 5mm de diamètre fait dans une gélose comprenant du sang, par exemple une gélose au sang de mouton 5% Columbia^{®}, BioRad^{®} (Marnes-la-Coquette, France). La boîte de gélose est incubée à 37°C et le diamètre d'hémolyse est mesuré après 24h.

Un mode de réalisation du procédé d'extraction éthanolique ou méthanolique à partir de solidage selon l'invention comprend les étapes suivantes :
- de macération de plante, par exemple 1kg de plante propre et sèche, dans de l'éthanol ou du méthanol à 50% dans de l'eau, par exemple 8 L d'éthanol (C₂H₅OH) à 50% 24h à 20°C ;
- d'évaporation de la phase éthanolique ou méthanolique, par exemple sous vide ;
- de rinçage, par exemple 2 fois, de la phase aqueuse restante avec du n-butanol, de préférence avec un rapport volumique extrait / n-butanol de 2:1 ;
- d'évaporation de la phase butanolique; puis
- d'élimination des tanins de la phase aqueuse, comme décrit précédemment lors d'un extraction aqueuse.

Dans ce mode de réalisation particulier, l'éthanol peut être remplacé par du méthanol, pour un procédé d'extraction méthanolique.

### Exemple 1 - Impact des saponines de S. virgaurea L. virgaurea sur les Candida

Les Demanderesses ont réalisé une étude sur deux souches de *C. albicans,* une souche de référence CP 4872 (fournie par l'Institut Pasteur) et une souche sauvage MG 001 (prélevée chez un patient).

Pour cela, les deux souches de *C. albicans* ont été cultivées pendant 24 h à 35°C, dans 100 *µ*L de milieu liquide RPMI (« Roswell Park Memorial Institute medium ») sans extrait de solidage ou bien dans 50 *µ*L de solution mère d'extrait de solidage obtenu par extraction aqueuse, cette solution mère étant diluée au 1/3 dans du RPMI. Les cultures de 24 h à 35°C en milieu liquide ont été ensemencées sur des boîtes de gélose (Sabouraud^{®}), incubées à 35°C pendant une nuit, puis laissées à 20°C (température ambiante) pendant 2 jours (J1 et J2). Chaque condition a été dupliquée 4 fois, correspondant aux tests n°1 à 4.

Après 1 jour (J1) à 20°C, le diamètre moyen des colonies de *C. albicans* a été mesuré par morphométrie, et le nombre moyen des colonies de *C*. *albicans* a été compté manuellement pour chaque puit à partir de dilutions 10⁻⁴ ou 10⁻².

Les résultats de ces mesures sont reportés dans les tableaux 1 à 3 ci-aprés. Les colonnes A correspondent aux cultures sans extrait de solidage, les colonnes B correspondent aux cultures en présence d'extrait de solidage dilué au 1/3. La ligne « Nombre » correspond au nombre de colonies dans chaque puit, tandis que la ligne « Diamètre » correspond au diamètre moyen des colonies, exprimé en mm, dans chaque puit.

Le diamètre moyen est calculé à partir des diamètres mesurés sur 100 colonies, sauf pour les tests n°3 et 4 avec CP4872 condition A où le nombre total de colonies était de 68 et 88 respectivement.

**Tableau 1 :**

| | *C. albicans* CP 4872 | | | | | | | |
|---|---|---|---|---|---|---|---|---|
| | Test n°1 | | Test n°2 | | Test n°3 | | Test n°4 | |
| | A | B | A | B | A | B | A | B |
| Nombre | 100 | 100 | 100 | 100 | 68 | 100 | 88 | 100 |
| Diamètre mm | 1,35 | 0,90 | 0,62 | 0,64 | 2,40 | 2,07 | 3,34 | 2,24 |
| Ecart type | 0,19 | 0,14 | 0,10 | 0,09 | 0,07 | 0,12 | 0,12 | 0,18 |

**Tableau 2 :**

| | *C. albicans* MG 001 | | | | | | | |
|---|---|---|---|---|---|---|---|---|
| | Test n°1 | | Test n°2 | | Test n°3 | | Test n°4 | |
| | A | B | A | B | A | B | A | B |
| Nombre | 100 | 100 | 100 | 100 | 100 | 100 | 100 | 100 |
| Diamètre mm | 1,20 | 1,05 | 0,86 | 0,80 | 2,58 | 1,72 | 2,86 | 1,74 |
| Ecart type | 0,13 | 0,12 | 0,12 | 0,11 | 0,10 | 0,05 | 0,20 | 0,07 |

**Tableau 3 : valeurs de significativité (« p-value ») associées aux comparaisons des valeurs de Diamètre précédentes entre condition A et condition B :**

| zip-value | *C. albicans* CP 4872 | *C. albicans* MG 001 |
|---|---|---|
| A/B | | |
| Test n°1 | 0,000000 | 0,000000 |
| Test n°2 | 0,12772 | 0,000034 |
| Test n°3 | 0,000000 | 0,000000 |
| Test n°4 | 0,000000 | 0,000000 |

Après une nuit à 35°C, la présence de colonies sur la gélose montre que les *Candida* ont poussé, même celles issues du milieu liquide avec solution mère. Donc les saponines selon l'invention n'ont pas un effet antifongique ni fongicide, en tous cas pas immédiat.

La culture en milieu liquide avec numération des unités formant colonie (ou CFU) n'a pas non plus montré d'effet fongicide ni fongistatique de la solution mère.

Après un jour (J1) de culture à 20°C, il n'y a pas eu de diminution significative du nombre de colonies issues du milieu liquide avec solution mère (condition B, p> 0,05) par rapport aux cultures issues du milieu RPMI seul (condition A) (Tableaux 1 et 2). Par ailleurs, le diamètre des colonies a significativement diminué pour la condition B (p<0,01). Pour le test n°2 avec la souche CP 4872, une différence significative n'est apparue qu'à J2 (p=0,007239). Donc la solution mère, et en particulier les saponines qu'elle contient, n'empêchent pas complètement mais ralentissent la croissance des champignons, par un effet différé et non pas immédiat. Elle crée un environnement défavorable à la croissance des deux souches de *Candida* testées et diminuent leur potentiel de croissance.

De plus, au microscope, l'aspect des colonies est différent. En effet, après un jour à 20°C, les cultures repiquées sur gélose sont observées au microscope. Les souches de *C*. *albicans* issues des conditions A ont développé de nombreux filaments pseudo-mycéliens, témoins d'une croissance active, tandis que les souches issues des conditions B se trouvent essentiellement sous forme de levures petites et rondes, d'aspect quiescent, sans ou avec très peu de filaments mycéliens, c'est-à-dire sous forme non virulente.

La figure 1 montre une vue au microscope de la souche *C*. *albicans* 4872 à J1 (Test n°3), issue, sur la photo de gauche, d'une culture sans solution mère (condition A), et, sur la photo de droite, issue d'une culture avec solution mère (condition B). La figure 2 montre une vue au microscope de la souche *C*. *albicans* MG 001 à J1 (Test n°3), issue, sur la photo de gauche, d'une culture sans solution mère, et, sur la photo de droite, issue d'une culture avec solution mère. La solution mère diluée au 1/3 semble plus active sur la souche sauvage MG 001 que sur la souche de référence 4872.

Les Demanderesses ont également observé que *Staphylococcus aureus, Escherichia coli* et *Streptococcus mutans* poussent au contact des puits contenant 50 *µ*l de solution mère diluée pure ou au 1/3. Cela montre que la solution mère n'a eu aucun effet inhibiteur sur ces souches bactériennes. Par ailleurs, un effet antifongiques similaire sur la morphologie des *Candida* est observé après 3 repiquages alternant incubation en présence de solution mère diluée au 1/3 (8 h à 35°C) et inoculation sur gélose Sabouraud^{®} (16 h à 35°C puis 24h à température ambiante pour la dernière boîte). Les repiquages simulent l'utilisation quotidienne du bain de bouche *in vivo.*

Ces observations montrent que les saponines selon l'invention, et en particulier les extraits de solidage comprenant des saponines et sans tanins, selon l'invention, permettent de maintenir les *Candida* sous une forme quiescente, non virulente, empêchant leur développement, et en particulier leur transformation dans une forme pathogène, mais sans les éradiquer. Ainsi, les saponines selon l'invention ont un effet doux et durable, non aggressif, créant un environnement qui défavorise la croissance des *Candida,* sans les lyser et sans les effets antimicrobiens dus aux tanins. Cela permet aux compositions selon l'invention d'être utilisées de manière prolongée, régulière, sans provoquer d'effets secondaires néfastes.

### Exemple 2 - Impact de différents extraits aqueux de solidages, Solidago virgaurea virgaurea et Solidago virgaurea minuta/alpestris, contenant des saponines sur C. albicans ATCC 10231

Les Demanderesses ont réalisé une autre étude sur neuf souches sauvages de *C*. *albicans* isolées au service de mycologie du centre hospitalier de Nice et une souche de référence ATCC 10231 (isolées au laboratoire de Microbiologie Orale de la Faculté de chirurgie dentaire cultivées sous deux formes, lisse et rugueuse), soit au total dix souches cultivées.

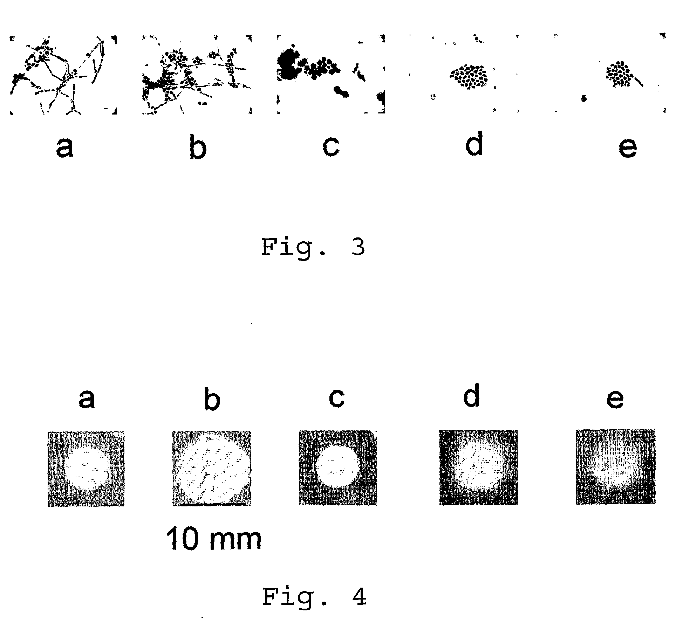

La transformation des levures *Candida* en filaments (figure 3) et le diamètre des colonies (figure 4) ont été observés après 72h de croissance des inoculums dans des boîtes de gélose contenant du Sabouraud^{®} / chloramphénicol puis dilués à 0,5 Mc Farland avec 100 *µ*L d'un milieu glutamine RPMI (« Roswell Park Memorial Institute medium ») supplémenté en glucose 1M (Invitrogen, Paisley, UK).

Tel qu'illustré à la figure 3a par marquage au cristal violet, l'incubation pendant 36h avec 50 *µ*L de chlorure de sodium à 0,9% dans le milieu provoque la transformation des levures de *Candida* en filaments mycéliens.

Le phénomène est encore plus prononcé à la figure 3b lorsque le milieu est acidifié à pH 4.

Dans les figures 3c, 3d et 3e, le chlorure de sodium est remplacé respectivement par 50 *µ*L de saponines d'écorces de Quillaja à 250 mg/mL, de décoctions d'extraits aqueux de *Solidago virgaurea virgaurea* ou de *Solidago virgaurea minuta*/*alpestris,* obtenues selon l'invention et ne contenant pas de tanins.

La figure 3c montre l'abolition de l'élongation des levures de *Candida* par les saponines à une concentration élevée de 250 mg/mL en induisant la lyse des cellules.

Le remplacement de la solution de saponines d'écorces de Quillaja par les décoctions d'extraits de solidages inhibent également la transformation des levures de *Candida* en filaments mycéliens tels qu'illustrés aux figures 3d et 3e.

Une telle inhibition est liée à la présence de saponines dans les extraits de solidages.

Cette inhibition a été observée pour les dix souches de *C. albicans* étudiées et illustrée dans la figure 3 sur *C***.** *albicans* ATCC 10231r (forme rugeuse).

Pour mesurer la taille des macro-colonies de *C. albicans,* les colonies obtenues selon les conditions de la figure 3 ont été repiquées dans des boîtes de géloses YEP contenant des géloses YEP (agar à 22 g/L avec extrait de levure à 11g/L, peptone à 22 g/L et glucose à 20 g/L modifiées ou non par l'ajout de milieu provoquant la défilamentation (saponines ou solution de solidages). Les boîtes temoin sans saponine ou décoction peuvent être acidifiées ou non par du tampon citrate. Pour obtenir les macrocolonies, les boîtes de gélose YEP précédentes où se sont développées les différentes souches de *C*. *albicans* sont respectivement piquées avec une pipette capillaire de 25 *µ*m de diamètre et inoculées dans des nouvelles boîtes de gélose YEP modifiées.

On observe la diminution de la taille des macro-colonies pour les conditions des figures 4c, 4d et 4e correspondant à une incubation avec respectivement des saponines d'écorces de Quillaja à 250 mg/mL, des décoctions d'extraits aqueux de *Solidago virgaurea virgaurea* ou de *Solidago virgaurea minuta*/*alpestris,* obtenues selon l'invention et ne contenant pas de tanins, par rapport à la condition contrôle de la figure 4b en milieu acide.

Le contrôle en milieu non acide illustré à la figure 4a ne montre pas une telle différence d'après cette expérience.

Une telle étude met en évidence l'importance du milieu acide dans la taille des macro-colonies et montre l'effet des saponines dans la diminution de la taille des macro-colonies.

Une telle diminution de la taille des colonies peut être liée à l'effet des saponines sur l'inhibition de la transformation des levures en filaments mycéliens des *C. albicans.*

Ces observations confirment que les saponines selon l'invention, et en particulier les extraits de solidages comprenant des saponines et sans tanins, selon l'invention, permettent de maintenir les *Candida* sous une forme quiescente, non virulente, empêchant leur développement, et en particulier leur transformation dans une forme pathogène, mais sans les éradiquer. Ainsi, les saponines selon l'invention ont un effet doux et durable, non aggressif, créant un environnement qui défavorise la croissance des *Candida,* sans les lyser et sans les effets antimicrobiens dus aux tanins. Cela permet aux compositions selon l'invention d'être utilisées de manière prolongée, régulière, sans provoquer d'effets secondaires néfastes.

## Revendications

1. Composition comprenant un extrait d'une plante solidage (*Solidago)* comprenant des saponines, **caractérisée en ce qu'**elle est substantiellement exempte de tanins, et **en ce que** les saponines n'ont pas subi de lyse alcaline dans le cas où l'extrait de plante consiste en des saponines purifiées.

2. Composition selon la revendication 1, **caractérisée en ce qu'**elle ne comprend pas de tanins.

3. Composition selon la revendication 1 ou 2, **caractérisée en ce qu'**elle se présente sous une forme de bain de bouche, dentifrice, gel, crème, lait, poudre, lotion, spray, savon ou ovules, et de préférence sous une forme de bain de bouche.

4. Composition comprenant un extrait d'une plante solidage *(Solidago)* comprenant des saponines et ne comprenant substantiellement pas de tanins, pour son utilisation cosmétique pour l'hygiène de la bouche, de la peau, des muqueuses, des ongles et/ou des cheveux.

5. Composition comprenant un extrait d'une plante solidage *(Solidago)* comprenant des saponines et ne comprenant substantiellement pas de tanins, pour son utilisation comme médicament.

6. Composition selon la revendication 5, pour son utilisation comme médicament dans le traitement des sécheresses cutanées et/ou des sécheresses des muqueuses, et en particulier des sécheresses buccales.

7. Composition selon la revendication 5, pour son utilisation comme médicament dans le traitement des mycoses, en particulier des mycoses cutanées, buccales ou ano-génitales.

8. Composition selon la revendication 5, pour son utilisation comme médicament dans le traitement des infections bucco-dentaires, notamment les caries, les parodontites, la langue noire et l'halithose liées à la sécheresse buccale.

9. Procédé d'obtention d'un extrait de plantes, de préférence solidages (*Solidago*)*,* ledit extrait comprenant des saponines et ne comprenant substantiellement pas de tanins, le procédé comprenant les étapes suivantes :
- d'extraction de saponines à partir de plantes, de préférence solidages (*Solidago*) ; puis
- d'élimination ciblée des tanins.

10. Procédé selon la revendication 9, **caractérisé en ce que** :
- l'étape d'extraction est une extraction aqueuse, éthanolique, méthanolique, une extraction au chloroforme ou encore une extraction à l'éther de pétrole ;
- l'étape d'extraction, dans le cas d'une extraction éthanolique, méthanolique, au chloroforme ou à l'éther de pétrole, est suivie d'une étape d'élimination de l'éthanol, du méthanol, du chloroforme ou de l'éther de pétrole, par exemple par évaporation ;
- l'étape d'élimination ciblée des tanins est effectuée par précipitation des tanins en présence de protéines, et de préférence en présence de caséine.

## Patentansprüche

1. Zusammensetzung, die einen Extrakt einer Goldrutenpflanze *(Solidago)* umfasst, der Saponine umfasst, **dadurch gekennzeichnet, dass** sie im Wesentlichen frei von Tanninen ist und dass die Saponine in dem Fall, in dem der Pflanzenextrakt aus gereinigten Saponinen besteht, keiner alkalischen Lyse unterzogen wurden.

2. Zusammensetzung nach Anspruch 1, **dadurch gekennzeichnet, dass** sie keine Tannine umfasst.

3. Zusammensetzung nach Anspruch 1 oder 2, **dadurch gekennzeichnet, dass** sie in einer Form eines Mundwassers, einer Zahnpasta, eines Gels, einer Creme, einer Milch, eines Puders, einer Lotion, eines Sprays, einer Seife oder eines Zäpfchens und vorzugsweise in einer Form eines Mundwassers vorliegt.

4. Zusammensetzung, die einen Extrakt einer Goldrutenpflanze *(Solidago)* umfasst, der Saponine umfasst und im Wesentlichen keine Tannine umfasst, für deren kosmetische Verwendung für die Mund-, Haut-, Schleimhaut-, Nagel- und/oder Haarpflege.

5. Zusammensetzung, die einen Extrakt einer Goldrutenpflanze *(Solidago)* umfasst, der Saponine umfasst und im Wesentlichen keine Tannine umfasst, für deren Verwendung als Arzneimittel.

6. Zusammensetzung nach Anspruch 5 für deren Verwendung als Arzneimittel bei der Behandlung von Hauttrockenheiten und/oder Schleimhauttrockenheiten und insbesondere Mundtrockenheiten.

7. Zusammensetzung nach Anspruch 5 für deren Verwendung als Arzneimittel bei der Behandlung von Pilzerkrankungen, insbesondere Haut-, Mund- oder anogenitalen Pilzerkrankungen.

8. Zusammensetzung nach Anspruch 5 für deren Verwendung als Arzneimittel bei der Behandlung von Mund- und Zahninfektionen, insbesondere Karies, Parodontitis, schwarzer Zunge und mit Mundtrockenheit assoziierter Halitosis.

9. Verfahren zum Erlangen eines Extrakts von Pflanzen, vorzugsweise Goldruten (*Solidago*)*,* wobei der besagte Extrakt Saponine umfasst und im Wesentlichen keine Tannine umfasst, wobei das Verfahren die folgenden Schritte umfasst:
- Extraktion von Saponinen aus Pflanzen, vorzugsweise Goldruten (*Solidago*); dann
- gezielte Beseitigung von Tanninen.

10. Verfahren nach Anspruch 9, **dadurch gekennzeichnet, dass**:
- der Extraktionsschritt eine wässrige, ethanolische, methanolische Extraktion, eine Extraktion mit Chloroform oder auch eine Extraktion mit Petrolether ist;
- auf den Extraktionsschritt im Fall einer ethanolischen, methanolischen Extraktion, einer Extraktion mit Chloroform oder mit Petrolether ein Schritt der Beseitigung des Ethanols, des Methanols, des Chloroforms oder des Petrolethers, beispielsweise durch Abdampfung folgt;
- der Schritt der gezielten Beseitigung von Tanninen durch Fällung der Tannine in Gegenwart von Proteinen und vorzugsweise in Gegenwart von Kasein durchgeführt wird.

## Claims

1. Composition containing a solidago *(Solidago)* plant extract containing saponins, **characterized in that** it is substantially free of tannins, and **in that** the saponins have not been subjected to alkaline lysis if the plant extract consists of purified saponins.

2. Composition according to claim 1, **characterized in that** it does not include tannins.

3. Composition according to claim 1 or 2, **characterized in that** it is in the form of a mouthwash, toothpaste, gel, cream, milk, powder, lotion, spray, soap or suppository, and preferably in the form of a mouthwash.

4. Composition containing a solidago *(Solidago)* plant extract containing saponins and containing substantially no tannins, for cosmetic use in hygiene of the mouth, skin, mucous membranes, nails and/or hair.

5. Composition containing a solidago *(Solidago)* plant extract containing saponins and containing substantially no tannins, for use as a drug.

6. Composition according to claim 5, for use as a drug in the treatment of dry skin and/or dry mucous membranes and in particular dry mouth.

7. Composition according to claim 5, for use as a drug in the treatment of mycoses, in particular skin, oral or anal-genital mycoses.

8. Composition according to claim 5, for use as a drug in the treatment of oral-dental infections, in particular cavities, periodontitis, black tongue and halitosis associated with dry mouth.

9. Process for obtaining a plant extract, preferably solidago *(Solidago),* said extract containing saponins and containing substantially no tannins, the process including the following steps:
- extraction of saponins from plants, preferably solidago (*Solidago*)*;* then
- targeted removal of tannins.

10. Process according to claim 9, **characterized in that**:
- the extraction step is an aqueous, ethanolic, methanolic, chloroform or petroleum ether extraction;
- the extraction step, in the case of an ethanolic, methanolic, chloroform or petroleum ether extraction, is followed by a step of removing ethanol, methanol, chloroform or petroleum ether, for example by evaporation;
- the step of targeted removal of the tannins is performed by precipitation of the tannins in the presence of proteins, and preferably in the presence of casein.
